# EUROPEAN PATENT SPECIFICATION

(11) **EP 2 179 692 B1**
(45) Date of publication and mention of the grant of the patent: **23.01.2013**
(21) Application number: 09173396.4
(22) Date of filing: 19.10.2009
(51) Int. Cl.: A61B 5/11

(54) **Patient positioning monitoring apparatus**
Vorrichtung zur Überwachung der Patientenpositionierung
Appareil de surveillance de la position du patient

(30) Priority: 21.10.2008 KR 20080103230
(43) Date of publication of application: 28.04.2010
(73) Proprietor: Humanscan Ltd, Ansan-si, Kyounggi-do (KR)
(72) Inventor: Hwang, Dae Sung, 152-793, Seoul (KR); Kim, Wan Cheol, 445-923, Kyounggi-do (KR)
(74) Representative: Neobard, William John

(56) References cited:
- US-A- 5 446 548
- US-A- 5 622 187
- US-A- 5 831 260
- US-A1- 2001 025 142
- US-A1- 2007 108 978

## Description

The present invention relates to the general field of position monitoring. Embodiments relate to a position guider of a patient position monitoring apparatus, to a guide marker of a patient position monitoring apparatus and to patient position monitoring apparatus, and more particularly, to a patient position monitoring apparatus capable of monitoring variations in the position of a patient.

There are many occasions when a patient should not be moved during medical treatment in a hospital because it is required to fix a body part of the patient to which the medical treatment is given. Accordingly, even a slight motion of the patient must be detected.

For example, ultrasonic therapy is required to radiate ultrasonic waves only to the affected part, and thus the motion of the patient must be continuously observed. Accordingly, a user (or a doctor) has to continuously watch the patient or marks the affected part of the patient, captures an image of the affected part using an optical camera and monitor the captured image in real time to detect a motion of the patient.

However, the aforementioned method requires the user to spend time because the user has to continuously monitor the patient. Furthermore, it is difficult to move the patient to the original position because the user determines the movement according to user's experience.

In US-A-2001/025142, movements of the patient or of a medical examination apparatus, for example of a C-arm, are acquired by sensors that supply corresponding signals. The sensors can be sensitive to magnetic fields and acquire the magnetic fields of magnet elements or the movement acquisition can ensue optically or acoustically. Outputs of the sensors are used to correct image data to eliminate effects of movement

In US-A- 5831260, a hybrid motion tracker captures motion of a person. Magnetic field sensors and optical sources are placed on a person, each located on different limbs. A fixed transmitter emits electromagnetic energy and infrared light is transmitted from the optical light sources to the fixed optical sensors. The magnetic field sensors sense the magnetic field and a computer calculates each sensor's position and orientation relative to the fixed transmitter. The optical system's Position Sensing Detectors measure the transmitted infrared light and the computer calculates the position and orientation of each optical light source. The position and orientation of each sensor is used to reconstruct the person's motion in real time, which is sent to a host computer. The computer utilizes the optical system which is more precise than the magnetic field system to compensate for the magnetic field system and thereby achieve higher accuracies. The system is a hybrid system using both magnetic fields and infrared light. By combining the two different technologies into one system, all of the advantages of the two systems by themselves are employed and none of their disadvantages become detrimental, in particular, accuracy and dynamic performance are enhanced over results obtained through sole use of a magnetically based system.

The invention is defined in the attached independent claim. Features of some embodiments are recited in the dependent claims.

When the patient moves, the patient can be returned to the original position by moving the patient such that the source emitted from the source irradiating parts reach the range of the source sensor.

Moreover, the interval of the source irradiating parts can be previously determined, and thus a treatment route and treatment area for the patient can be calculated through the first controller included in the position guider to provide convenience and accuracy during medical treatment.

In the drawings:
FIG. 1 illustrates a patient position monitoring apparatus according to an embodiment of the present invention;
FIG. 2 is a block diagram of ultrasonic therapy equipment including the patient position monitoring apparatus illustrated in FIG. 1;
FIG. 3 is a cross-sectional view of a strap and a guide marker of the patient position monitoring apparatus illustrated in FIG. 1 according to an embodiment of the present invention; and
FIGS. 4a, 4b, 4c and 4d illustrate various arrangements of source emitting parts of the patient position monitoring apparatus according to the present invention.

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those skilled in the art. Like reference numerals in the drawings denote like elements, and thus their description will be omitted.

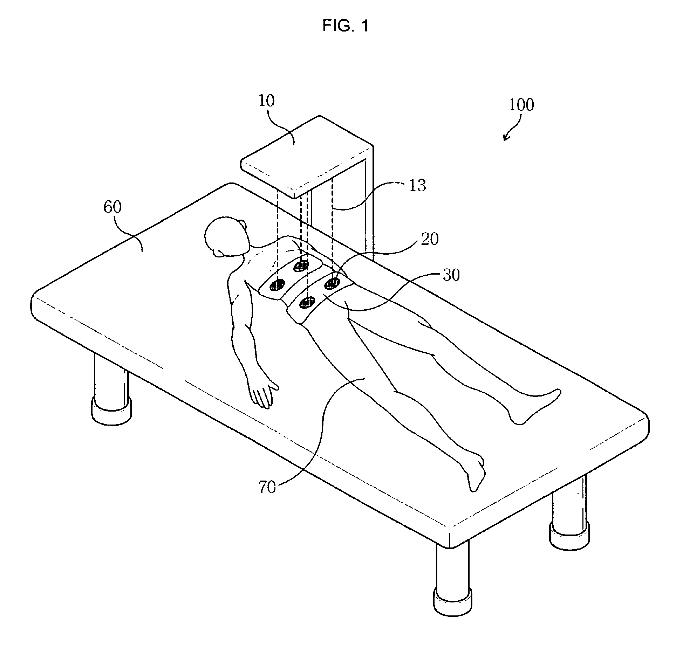

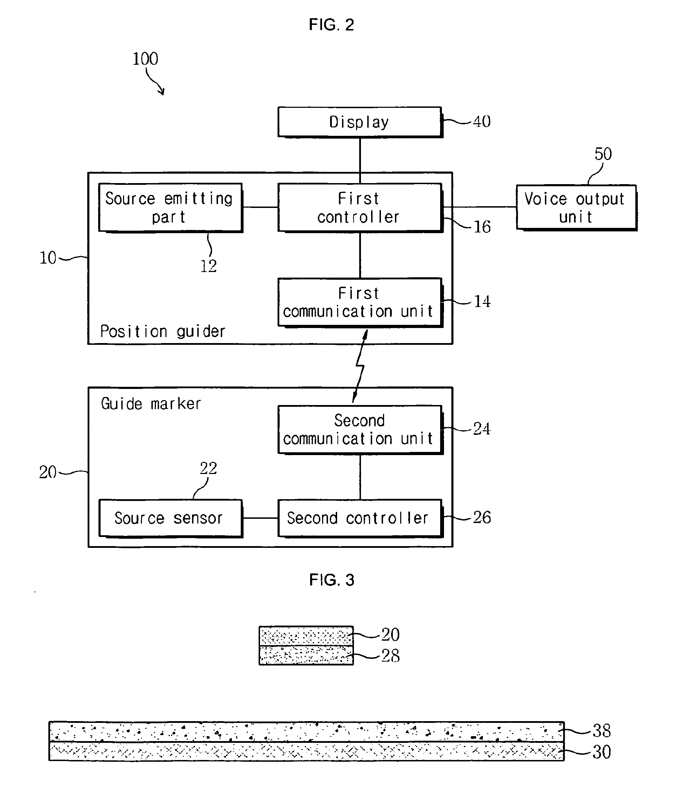

A patient position monitoring apparatus according to an embodiment of the present invention will now be explained with reference to FIGS. 1, 2 and 3. FIG. 1 illustrates the patient position monitoring apparatus 100 according to an embodiment of the present invention, FIG. 2 is a block diagram of ultrasonic therapy equipment including the patient position monitoring apparatus according to an embodiment of the present invention, and FIG. 3 is a cross-sectional view of a strap and a guide marker of the patient position monitoring apparatus according to the present invention.

The patient position monitoring apparatus 100 monitors the position of a patient 70 and outputs a warning signal if the position of the patient 70 is changed during medical treatment. The patient position monitoring apparatus 100 according to the current embodiment of the invention includes a position guider 10, a plurality of guide markers 20 and a strap 30. The patient position monitoring apparatus 100 may further include output units. Here, the output units include a display 40 and a voice output unit 50. The output units may further include a lamp which outputs a warning signal as flickering light.

The display 40 displays a menu of various functions executed in the patient position monitoring apparatus 100. The display 40 may indicate points to which the guide markers 20 are attached or a treatment route of the patient 70. A liquid crystal display (LCD) or a touch screen may be used as the display 40. The touch screen functions as both a display device and an input device.

The voice output unit 50 outputs a voice under the control of the position guider 10. Particularly, the voice output unit 50 receives the warning signal from the position guider 10 and outputs the warning signal as a voice.

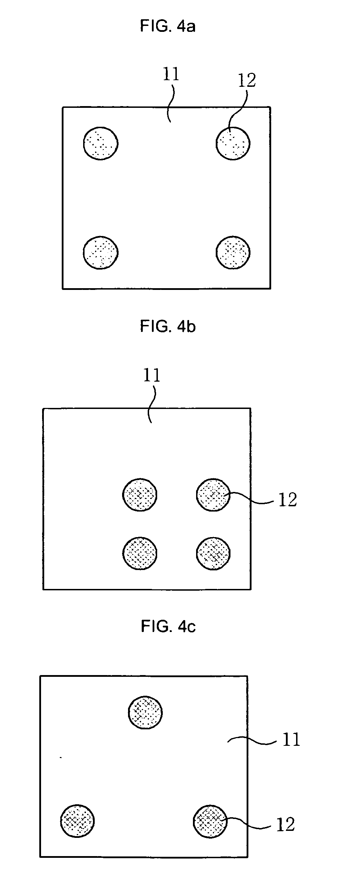

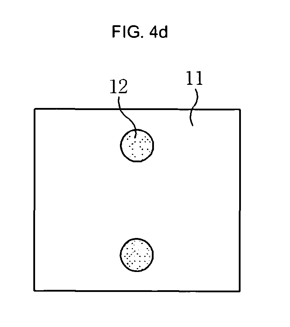

The position guider 10 includes source emitting parts 12, a first communication unit 14 and a first controller 16. The source emitting parts 12 are located above the patient to which the plurality of guide markers 20 are attached and emit a source to the guide markers 20. The number of source emitting parts 12 corresponds to the number of guide markers 20. The source emitting parts 12 are formed on a source emitting plate 11 (refer to FIG. 4) and emit one of a laser, LED light, ultrasonic waves and infrared rays. For example, when the source emitting parts 12 emit laser beams 13, the laser beams 13 arrive at the patient 70 located under the source emitting parts 12 and are indicated on the strap 30 attached to the affected part of the patient 70. The source emitting parts 12 will be described later with reference to FIG. 4.

The first communication unit 14 performs communication with the guide markers 20. Here, wired or wireless communication can be used.

The first controller 16 corresponds to a microprocessor that controls the overall operation of the position guider 10. The first controller 16 receives information on the sensed source from the guide markers 20 and determines whether the position of the patient 70 is changed.

Further, the position guider 10 illustrated in FIG. 1 has the source emitting parts 12 formed on the side facing the patient 70. However, the shape of the position guider 10 is not limited thereto. The position guider 10 may be fixed to a therapy table 60 or a movable supporting table.

Each of the guide markers 20 includes a source sensor 22, a second communication unit 24 and a second controller 26. The source sensor 22 senses the source emitted from the corresponding source emitting part 12. The source sensor 22 may be placed opposite to the source emitting part 12 to stably sense the source emitted from the source emitting part 12. The source sensor 22 is selected according to the type of the source emitted from the source emitting part 12 and includes one of a laser sensor, an LED sensor, an ultrasonic sensor and an infrared sensor. For example, when a laser is emitted from the source emitting part 12, the laser sensor is used as the source sensor 22.

The second communication unit 24 performs communication with the position guider 10. Here, wired or wireless communication may be used.

Further, the second controller 26 corresponds to a microprocessor which controls the overall operation of the guide markers 20. The second controller 26 transmits information sensed by the source sensor 22 to the position guider 10 through the second communication unit 24. The first communication unit 14 and the second communication unit 24 are connected to each other through a wired or wireless method.

The first controller 16 of the position guider 10 receives the information sensed by the source sensor 22, determines whether the source emitted from the source emitting part 12 is out of the range of the source sensor 22 through the received information and judges that the position of the patient 70 is changed if when the source is out of the range of the source sensor 22. When it is determined that the position of the patient 70 is changed, the first controller 16 can output a warning signal through the voice output unit 16. Further, the first controller 16 can output the warning signal through the display 40. When the position of the patient 70 is changed and thus the warning signal is output through the voice output unit 50 or the display 40, the user can recognize the change of the patient position and move the patient 70 to the original position such that the source irradiated from the source emitting part 12 reaches the range of the source sensor 22.

The strap 30 is used to attach the guide markers 20 to the patient 70 and is located on the affected part of the patient 70. The strap 30 includes a first adhesive part 38 formed on one side thereof. The strap 30 may cover the body of the patient 70 including the affected part or only the affected part. The first adhesive part 38 is formed to attach the guide markers 20 to the strap 30. A Velcro^{®} tape or a metal fiber can be used as the first adhesive part 38.

Each of the guide markers 20 includes a second adhesive part 28 formed on one side thereof. The second adhesive part 28 is attached to the first adhesive part 38 of the strap 30. The second adhesive part 28 may use a Velcro^{®} tape when the first adhesive part 38 is a Velcro^{®} tape and use a magnet when the first adhesive part 38 is a metal fiber. The first adhesive part 38 and the second adhesive part 28 are not limited to the Velcro^{®} tape, metal fiber and magnet and can use various methods which can attach the first and second adhesive parts 38 and 28 to each other.

Although the patient position monitoring apparatus 100 of the current embodiment of the invention includes the display 40 and the voice output unit 50 which are separated from the position guider 10, the present invention is not limited thereto. For example, the position guider 10 may include the display 40 or the voice output unit 50.

The source emitting parts 12 according to an embodiment of the present invention will now be explained with reference to FIGS. 4a, 4b, 4c and 4d. FIGS. 4a, 4b, 4c and 4d illustrate various shapes of the source emitting parts of the patient position monitoring apparatus of the present invention.

The plurality of source emitting parts 12 are formed on the source emitting plate 11. The source emitting parts 12 are connected to the position guider 10 through a wired or wireless method and emit a source. The source emitting parts 12 may be respectively formed at four corners of the source emitting plate 11, as shown in FIG. 4a, and the interval or positions of the source emitting parts 12 may be varied, as shown in FIG. 4b. Further, the source emitting parts 12 may be arranged in a triangular shape, as shown in FIG. 4c, and two source emitting parts 12 may be formed in a row, as shown in FIG. 4d.

Though FIGS. 4a, 4b, 4c and 4d show the source emitting parts 12 in four arrangement forms, the present invention is not limited thereto.

Furthermore, the patient position monitoring apparatus 100 may include a cover for covering one of the plurality of source emitting parts 12 to reduce the number of source emitting parts 12.

The interval of the source emitting parts 12 formed on the source emitting plate 11 can be determined in advance, and thus the first controller 16 can calculate a treatment route or a treatment area for the patient through the previously determined interval of the sources 13 corresponding to the source emitting parts 12.

Moreover, the source emitting parts 12 may be designed such that the source emitting parts 12 can move on the source emitting plate 11. In addition, the source emitting parts 12 may be connected to a flexible rail without using the source emitting plate 11 to change the positions and number of the source emitting parts 12. The source emitting parts 12 can be formed in various manners.

While the present invention has been particularly shown and described with reference to exemplary embodiments thereof, it will be understood by those of ordinary skill in the art that various changes in forms and details may be made therein without departing from the scope of the present invention as defined by the following claims.

## Claims

1. A patient position monitoring apparatus (100) comprising:
a position guider (10) for emitting a source; and
a plurality of guide markers (20) for sensing the source emitted from the position guider (10) and transmitting sensed information to the position guider (10), the guide markers (20) being attachable to a patient; **characterized in that**
the position guider (10) is configured to determine through the sensed information whether positions of the guide markers are changed_{;} and
the apparatus further comprises a strap (30) that has a first adhesive part (38) for attaching the guide markers (20) to the strap (30), which is formed on one side of the strap (30), the strap (30) being configured to be located on an affected part of the patient.

2. The patient position monitoring apparatus of claim 1, wherein the position guider (10) comprises:
a plurality of source emitting parts (12) configured to be located above the patient and for emitting the source to the guide markers (20);
a first communication unit (14) for performing communication with the guide markers (20); and
a first controller (16) configured to receive information sensed by the guide markers (20) from the guide markers (20) through the first communication unit (14) and to determine whether the position of the patient is changed.

3. The patient position monitoring apparatus of claim 2, wherein the position guider (10) further comprises a source emitting plate (11) on which the source emitting parts (12) are arranged.

4. The patient position monitoring apparatus of claim 2, wherein the source emitting parts (12) are arranged to emit one of a laser, LED light, ultrasonic waves and infrared rays.

5. The patient position monitoring apparatus of claim 1, wherein each of the guide markers (20) comprises:
a source sensor (22) for sensing the source emitted from the position guider (10);
a second communication unit (24) for performing communication with the position guider (10); and
a second controller (26) for transmitting information sensed by the source sensor (22) to the position guider (10) through the second communication unit (24).

6. The patient position monitoring apparatus of claim 5 wherein the source sensor includes one of a laser sensor, an LED sensor, an ultrasonic sensor and an infrared sensor.

7. The patient position monitoring apparatus of claim 1, wherein the first adhesive part (28) corresponds to one of a Velcro® tape and a metal fiber.

8. The patient position monitoring apparatus of claim 8, wherein a second adhesive part (28) for attaching each guide marker (20) to the strap (30) is formed on the bottom face of each guide marker (20)/and the second adhesive part (28) corresponds to one of a Velcro® tape and a magnet.

9. The patient position monitoring apparatus of claim 1, further comprising an output unit for receiving a warning signal for warning a user that the positions of the guide markers (20) are changed from the position guider (10) and outputting the warning signal.

10. The patient position monitoring apparatus of claim 2, wherein each of the guide markers (20) comprises:
a source sensor (22) sensing the source emitted from the position guider (10);
a second communication unit (24) for performing communication with the position guider (10); and
a second controller (26) for transmitting information sensed by the source sensor (22) to the position guider (10) through the second communication unit (24).

11. The patient position monitoring apparatus of claim 12, wherein the position guider (10) is configured to judge that the position of the patient is changed if it is determined that the source emitted from the source emitting parts (12) is out of the range of the source sensor (22) through the sensed information.

12. The patient position monitoring apparatus of claim 13, further comprising an output unit for receiving a warning signal for warning a user of a change in the patient position from the position guider (10) and outputting the warning signal.

## Patentansprüche

1. Patientenpositionsüberwachungsvorrichtung (100) mit:
einem Positionsführer (10) zum Emittieren einer Quelle; und
einer Mehrzahl von Führungsmarkierungen (20) zum Abtasten der vom Positionsführer (10) emittierten Quelle und zum Senden von abgetasteten Informationen an den Positionsführer (10), wobei die Führungsmarkierungen (20) an einem Patienten anbringbar sind; **dadurch gekennzeichnet, dass**
der Positionsführer (10) konfiguriert ist, um über die abgetasteten Informationen festzustellen, ob sich Positionen der Führungsmarkierungen verändert haben; und
die Vorrichtung weiters einen Gurt (30) mit einem ersten haftenden Teil (38) zum Anbringen der Führungsmarkierungen (20) am Gurt (30) aufweist, der auf einer Seite des Gurts (30) ausgebildet ist, wobei der Gurt (30) konfiguriert ist, um auf einem betroffenen Teil des Patienten angeordnet zu werden.

2. Patientenpositionsüberwachungsvorrichtung nach Anspruch 1, wobei der Positionsführer (10) aufweist:
eine Mehrzahl von Quellenemissionsteilen (12), die konfiguriert sind, um zum Emittieren der Quelle an die Führungsmarkierungen (20) über dem Patienten angeordnet zu werden;
eine erste Kommunikationseinheit (14) zur Durchführung einer Kommunikation mit den Führungsmarkierungen (20); und
eine erste Steuerung (16), die konfiguriert ist, von den Führungsmarkierungen (20) abgetastete Informationen von den Führungsmarkierungen (20) über die erste Kommunikationseinheit (14) zu empfangen und festzustellen, ob sich die Position des Patienten verändert hat.

3. Patientenpositionsüberwachungsvorrichtung nach Anspruch 2, wobei der Positionsführer (10) weiters eine Quellenemissionsplatte (11) aufweist, auf der die Quellenemissionsteile (12) angeordnet sind.

4. Patientenpositionsüberwachungsvorrichtung nach Anspruch 2, wobei die Quellenemissionsteile (12) eingerichtet sind, einen Laser, ein LED-Licht, Ultraschallwellen oder Infrarotstrahlen zu emittieren.

5. Patientenpositionsüberwachungsvorrichtung nach Anspruch 1, wobei jede Führungsmarkierung (20) aufweist:
einen Quellensensor (22) zum Abtasten der vom Positionsführer (10) emittierten Quelle;
eine zweite Kommunikationseinheit (24) zur Durchführung einer Kommunikation mit dem Positionsführer (10); und
eine zweite Steuerung (26) zum Senden von vom Quellensensor (22) abgetasteten Informationen an den Positionsführer (10) über die zweite Kommunikationseinheit (24).

6. Patientenpositionsüberwachungsvorrichtung nach Anspruch 5, wobei der Quellensensor einen Lasersensor, einen LED-Sensor, einen Ultraschallsensor oder einen Infrarotsensor aufweist.

7. Patientenpositionsüberwachungsvorrichtung nach Anspruch 1, wobei der erste haftende Teil (38) einem Velcro®-Band oder einer Metallfaser entspricht.

8. Patientenpositionsüberwachungsvorrichtung nach Anspruch 8, wobei ein zweiter haftender Teil (28) zum Anbringen jeder Führungsmarkierung (20) am Gurt (30) auf der Unterseite jeder Führungsmarkierung (20) ausgebildet ist und der zweite haftende Teil (28) einem Velcro®-Band oder einem Magnet entspricht.

9. Patientenpositionsüberwachungsvorrichtung nach Anspruch 1, weiters mit einer Ausgabeeinheit zum Empfangen eines Warnsignals vom Positionsführer (10) zum Warnen eines Benutzers, dass sich die Positionen der Führungsmarkierungen (20) verändert haben, und zum Ausgeben des Warnsignals.

10. Patientenpositionsüberwachungsvorrichtung nach Anspruch 2, wobei jede Führungsmarkierung (20) aufweist:
einen Quellensensor (22) zum Abtasten der vom Positionsführer (10) emittierten Quelle;
eine zweite Kommunikationseinheit (24) zur Durchführung einer Kommunikation mit dem Positionsführer (10); und
eine zweite Steuerung (26) zum Senden von vom Quellensensor (22) abgetasteten Informationen an den Positionsführer (10) über die zweite Kommunikationseinheit (24).

11. Patientenpositionsüberwachungsvorrichtung nach Anspruch 12, wobei der Positionsführer (10) konfiguriert ist, um zu beurteilen, dass sich die Position des Patienten verändert hat, wenn durch die abgetasteten Informationen festgestellt wird, dass sich die von den Quellenemissionsteilen (12) emittierte Quelle außerhalb des Bereichs des Quellensensors (22) befindet.

12. Patientenpositionsüberwachungsvorrichtung nach Anspruch 13, weiters mit einer Ausgabeeinheit zum Empfangen eines Warnsignals zum Warnen eines Benutzers vor einer Änderung der Patientenposition vom Positionsführer (10) und zum Ausgeben des Warnsignals.

## Revendications

1. Appareil de surveillance de position de patient (100) comprenant :
un guide de position (10) pour émettre une source ; et
une pluralité de marqueurs de guidage (20) pour détecter la source émise du guide de position (10) et transmettre des informations détectées au guide de position (10), les marqueurs de guidage (20) pouvant être attachés à un patient ; **caractérisé en ce que**
le guide de position (10) est configuré pour déterminer, par le biais des informations détectées, si des positions des marqueurs de guidage ont changé ; et
l'appareil comprend en outre une sangle (30) ayant une première partie adhésive (38) pour attacher les marqueurs de guidage (20) à la sangle (30), qui est formée sur un côté de la sangle (3), la sangle (30) étant configurée pour être située sur une partie affectée du patient.

2. Appareil de surveillance de position de patient selon la revendication 1, dans lequel le guide de position (10) comprend :
une pluralité de parties d'émission de source (12) configurées pour être situées au-dessus du patient et pour émettre la source aux marqueurs de guidage (20) ;
une première unité de communication (14) pour effectuer une communication avec les marqueurs de guidage (20) ; et
un premier organe de commande (16) configuré pour recevoir des informations détectées par les marqueurs de guidage (20) à partir des marqueurs de guidage (20) par le biais de la première unité de communication (14) et pour déterminer si la position du patient a changé.

3. Appareil de surveillance de position de patient selon la revendication 2, dans lequel le guide de position (10) comprend en outre une plaque d'émission de source (11) sur laquelle sont agencées les parties d'émission de source (12).

4. Appareil de surveillance de position de patient selon la revendication 2, dans lequel les parties d'émission de source (12) sont agencées pour émettre l'un d'un laser, d'une lumière LED, d'ondes ultrasoniques et de rayons infrarouges.

5. Appareil de surveillance de position de patient selon la revendication 1, dans lequel chacun des marqueurs de guidage (20) comprend :
un capteur de source (22) pour détecter la source émise du guide de position (10) ;
une deuxième unité de communication (24) pour effectuer une communication avec le guide de position (10) ; et
un deuxième organe de commande (26) pour transmettre des informations détectées par le capteur de source (22) au guide de position (10) par le biais de la deuxième unité de communication (24).

6. Appareil de surveillance de position de patient selon la revendication 5, dans lequel le capteur de source comprend l'un d'un capteur laser, d'un capteur LED, d'un capteur d'ultrasons et d'un capteur d'infrarouges.

7. Appareil de surveillance de position de patient selon la revendication 1, dans lequel la première partie adhésive (38) correspond à l'une d'une bande Velcro^{®} et d'une fibre de métal.

8. Appareil de surveillance de position de patient selon la revendication 8, dans lequel une deuxième partie adhésive (28) pour attacher chaque marqueur de guidage (20) à la sangle (30) est formée sur la face inférieure de chaque marqueur de guidage (20) et la deuxième partie adhésive (28) correspond à l'un d'une bande Velcro^{®} et d'un aimant.

9. Appareil de surveillance de position de patient selon la revendication 1, comprenant en outre une unité de sortie pour recevoir un signal d'avertissement afin d'avertir un utilisateur que les positions des marqueurs de guidage (20) ont changé à partir du guide de position (10) et délivrer le signal d'avertissement.

10. Appareil de surveillance de position de patient selon la revendication 2, dans lequel chacun des marqueurs de guidage (20) comprend :
un capteur de source (22) détectant la source émise du guide de position (10) ;
une deuxième unité de communication (24) pour effectuer une communication avec le guide de position (10) ; et
un deuxième organe de commande (26) pour transmettre des informations détectées par le capteur de source (22) au guide de position (10) par le biais de la deuxième unité de communication (24).

11. Appareil de surveillance de position de patient selon la revendication 10, dans lequel le guide de position (10) est configuré pour juger que la position du patient a changé s'il est déterminé que la source émise des parties d'émission de source (12) est hors de portée du capteur de source (22) par le biais des informations détectées.

12. Appareil de surveillance de position de patient selon la revendication 13, comprenant en outre une unité de sortie pour recevoir un signal d'avertissement pour avertir un utilisateur d'un changement de la position de patient à partir du guide de position (10) et délivrer le signal d'avertissement.
